(19) Europäisches Patentamt
European Patent Office
Office européen des brevets

(11) Numéro de publication: **0 064 012**
**B1**

(12) # FASCICULE DE BREVET EUROPÉEN

(45) Date de publication du fascicule du brevet: **23.07.86**

(21) Numéro de dépôt: **82400770.2**

(22) Date de dépôt: **28.04.82**

(51) Int. Cl.[4]: **C 07 H 15/04**, C 07 H 15/20, A 61 K 31/70 // C07H11/00, C07H3/04

(54) **Nouveaux disaccharides formés de motifs à structure glucosamine et acide uronique, leur préparation et leurs applications biologiques.**

(30) Priorité: **28.04.81 FR 8108472**

(43) Date de publication de la demande:
**03.11.82 Bulletin 82/44**

(45) Mention de la délivrance du brevet:
**23.07.86 Bulletin 86/30**

(84) Etats contractants désignés:
**AT BE CH DE FR GB IT LI LU NL SE**

(56) Documents cité:
**EP-A-0 014 184**
**EP-A-0 027 089**
**EP-A-0 048 231**

**CHEMICAL ABSTRACTS, vol. 55, no. 26, 25 décembre 1961, colonne 27533d-e, COLUMBUS OHIO (US), S.A. BARKER et al.: "Some enzyme transfer reactions involving 2-acetamido-2-deoxy-D-glucose"**
**CARBOHYDRATE RESEARCH, vol. 78, 1980, Elsevier Scientific Publishing Company, AMSTERDAM (NL), U. KLEIN et al.: "A 3H-labelled trisaccharide from heparin as substrate for acetyl-CoA: 2-amino-2-deoxy-alpha-d-glucoside n-acetyl-transferase", pages 249-256**

(73) Titulaire: **CHOAY S.A., 48, Avenue Théophile-Gautier, F-75782 Paris Cédex 16 (FR)**

(72) Inventeur: **Petitou, Maurice, 27, rue du Javelot - Appt. 201, F-75645 Paris Cedex 13 (FR)**
Inventeur: **Sinay, Pierre, 5, rue Jacques Monod, F-45100 Orleans (FR)**
Inventeur: **Choay, Jean, 21, rue Saint- Guillaume, F-75007 Paris (FR)**
Inventeur: **Lormeau, Jean- Claude, 1, rue Joseph Delattre, F-76150 Maromme (FR)**

(74) Mandataire: **Gutmann, Ernest, S.C. Ernest Gutmann - Yves Plasseraud 67, boulevard Haussmann, F-75008 Paris (FR)**

**EP 0 064 012 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

L'invention est relative à de nouveaux disaccharides possédant, notamment, des propriétés biologiques, formés de motifs à structure respectivement glucosamine et acide uronique.

Elle vise, plus spécialement, des disaccharides 1,4α, formés de motifs à structure D-glucosamine et acide glucuronique.

Elle vise également leur préparation ainsi que leurs applications biologiques et biochimiques, notamment en tant que principe actif de médicaments.

De nombreux oligosaccharides ont déjà été décrits.

Ainsi, dans le vol. 55, No. 26, 1961, colonne 27533, d.e. des Chemical Abstracts, on rapporte l'obtention par BARKER et al par réaction enzymatique de composés renfermant du 2-acétamido-2-déoxy-D-glucose.

Dans Carbohydrate Research, 78 (1980) 249—256, on décrit l'obtention à partir d'héparine d'un trisaccharide utilisable comme substrat d'enzyme, de structure 2-amino-2-déoxy-α-D-glucosyl-(1→4)-acide aldosyluronique-(1→4)-2,5-anhydro-D-[1-$^3$H]mannitol.

Dans la demande de brevet EP No. 014184, on décrit des fragments d'héparine obtenus par dépolymérisation chimique de l'héparine, suivie d'une purification à l'aide d'antithrombine III. Il s'agit de fragments renfermant 14 à 18 motifs sucre dans lesquels le composant principal est constitué par le motif disaccharidique L-iduronosyl-2-O-sulfate-N-sulpho-U-glucosamine-6-O-sulphate et le motif uronique se trouve en position 3—5 à partir de l'extrêmité non réductrice.

Des octasaccharides et des oligosaccharides inférieurs, obtenus par dépolymérisation chimique ou enzymatique de l'héparine sont, en outre, décrits dans la demande EP No. 027089 de la Demanderesse.

Dans la demande EP No. 048 231, publiée entre la date de priorité et la date de dépôt de cette demande, on décrit des oligosaccharides contenant 4 à 8 motifs saccharidiques obtenus à partir d'héparine.

L'invention vise de nouveaux disaccharides répondant à la formule (I):

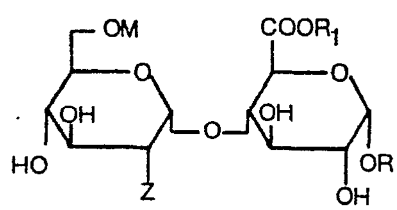

(I)

dans laquelle:

— *Z* représente un groupe azide $N_3$ ou un groupe comportant une fonction amine, pouvant être obtenue selon les réactions classiques de chimie organique, à partir du groupe azide;

— *M* représente un atome d'hydrogène ou un groupe $—SO_3M_1$ dans lequel $M_1$ représente un cation organique ou minéral, et, dans ce dernier cas, en particulier un métal alcalin, ou représente un groupe acétyle;

— *R* représente un radical alcoyle de 1 à 4 atomes de carbone, en particulier, un radical méthyle, ou encore aryle, et

— $R_1$ représente un atome d'hydrogène, un radical alcoyle comportant de 1 à 4 atomes de carbone, en particulier, un radical méthyle, ou un métal, en particulier, un métal alcalin.

Ces disaccharides possèdent avantageusement la structure demotifs constitutifs de chaînes d'héparine. Ils comportent, en effet, sur le motif glucosamine un groupe azoté en position 2 et un groupe alcool primaire avantageusement sulfaté en position 6, et sur le motif acide glucuronique un groupement fonctionnel acide ou dérivé d'un acide en position 6.

Des disaccharides préférés répondent à la formule (II):

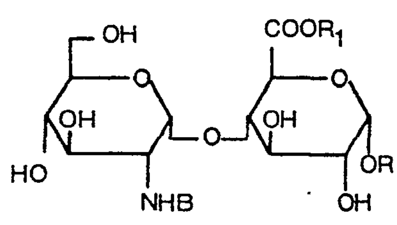

(II)

D'autres disaccharides spécialement préférés répondent à la formule (III):

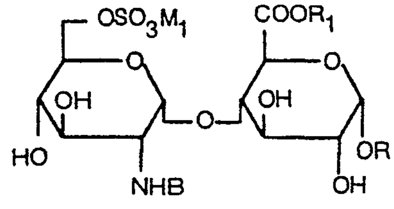

(III)

Dans ces formules *B, R, $R_1$* et *$M_1$* présentent les significations données ci-dessus, *$M_1$* représentant avantageusement un métal alcalin, en particulier, du sodium.

Dans des familles préférées de ces disaccharides II et III, le substituant *B* représente un groupe acétyle. Il s'agit donc de familles comportant un motif N-acétyl D-glucosamine. D'autres familles de disaccharides de l'invention comportent comme motifs glucosamine, des N-sulfate D-glucosamine. Dans ces familles, *B* représente un groupe $—SO_3M_2$ dans lequel *$M_2$*, identique ou différent de *$M_1$*, représente un métal, en particulier un métal alcalin, plus spécialement du sodium.

Des produits de ces familles comportent des substituants *$R_1$* et *R* identiques et représentant un groupe alcoyle, en particulier un groupe méthyle ou aryle.

Dans d'autres produits préférés, ces substituants sont différents et représentent respectivement un métal alcalin, en particulier, du sodium (*$R_1$*) et un radical alcoyle, en particulier méthyle (*R*).

Un produit de ce type plus spécialement préféré répond à la formule (IV):

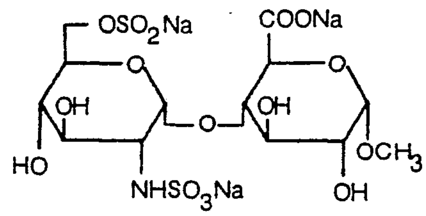

(IV)

L'étude pharmacologique des disaccharides de l'invention a montré qu'ils possèdent, notamment, des propriétés biologiques leur permettant de contrôler, de manière spécifique, certaines étapes de la coagulation sanguine.

Ces produits se révèlent notamment doués d'une activité d'inhibition sélective du facteur X activé ou facteur Xa du sang, et ce, plus particulièrement en ce qui concerne les disaccharides de formule III, plus spécialement ceux comportant un motif N-sulfate D-glucosamine.

Ils constituent, à cet égard, des réactifs de référence permettant des mesures comparatives visant à apprécier l'activité relative d'inhibition du facteur Xa les substances à l'étude. Le disaccharide de formule IV présente, par exemple, une activité anti-Xa, mesurée selon le test de Yin-Wessler, de l'ordre de 1 000 à 2 000 u/g.

Le test de Yin et Wessler, qui permet de mesurer une activité représentative plus spécialement de l'aptitude de produits à potentialiser l'inhibition du facteur Xa du sang par l'antithrombine III, ou AT III, est décrit par ces auteurs dans J. Lab. Clin, Med. 1976, 81, pp 298 à 300.

Ces disaccharides peuvent être avantageusement utilisés en tant que principe actif de médicament pour le contrôle de la coagulation sanguine in vivo, chez l'homme ou l'animal soumis à des risques d'hypercoagulabilité, tels que ceux induits par des interventions chirurgicales, des processus athéromateux, des perturbations des mécanismes de la coagulation par des activateurs bactériens ou enzymatiques, etc., comme conséquence de la libération dans l'organisme de thromboplastines, par exemple de thromboplastine tissulaire.

L'intérêt de ces disaccharides est encore accru en raison de leur innocuité.

L'invention concerne donc également les compositions pharmaceutiques dans lesquelles ces composés sont associés en quantité efficace à un véhicule pharmaceutique.

Elle concerne notamment les compositions dans lequelles le véhicule pharmaceutique est approprié pour l'administration par voie orale. Des formes d'administration de l'invention appropriées par l'administration par voie orale peuvent être avantageusement des gélules gastrorésistantes, des comprimés ou tablettes, des pilules, ou encore présentées sous forme de liposomes.

D'autres compositions pharmaceutiques comprennent ces disaccharides en association avec les excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables.

Afin d'illustrer l'invention, on indique, ci-après un exemple de posologie utilisable chez l'homme:cette posologie comprend, par exemple, l'administration au patient de 50 mg à 2 g disaccharide, deux ou trois fois par jour. Ces doses peuvent être naturellement ajustées pour chaque patient en fonction des résultats et des analyses de sang effectuées auparavant, la nature des affections dont il souffre et, d'une manière générale, son état de santé.

L'invention se rapporte également à l'application des disaccharides de l'invention, à la constitution des réactifs biologiques, utilizables en laboratoires, notamment comme éléments de comparaison pour l'étude d'autres substances dont on souhaite tester l'activité anticoagulante, notamment au niveau de l'inhibition du facteur Xa.

L'invention vise également un procédé de synthèse des disaccharides définis ci-dessus.

Selon ce procédé, on met en oeuvre deux monosaccharides permettant, a) de par leur structure, b) de

par la nature de leurs groupements réactifs, c) de par la nature de leurs groupements de bloquage, la réalisation de la stratégie de synthèse selon l'invention.

Ces groupements de bloquage sont avantageusement en nombre supérieurs à 1, ils permettent d'introduire après débloquage, des groupements fonctionnels ainsi que des groupements substitués spécifiques tels qu'existant sur la structure des disaccharides de l'invention, ils sont compatibles avec les groupements réactifs, ils sont capables de subir des débloquages séquentiels, compatibles entre eux et compatibles avec les substituants introduits à chaque étape.

On réalise une réaction de condensation entre ces deux monosaccharides, afin d'établir la liaison 1,4α recherchée dans des conditions compatibles avec les différents substituants des monosaccharides, puis on procède par étapes successives à l'introduction des groupements fonctionnels et des substituants désirés pour un disaccharide donné par mise en jeu de réactions spécifiques correspondantes.

Les monosaccharides mis en oeuvre répondent respectivement aux formules V et VI:

(V) 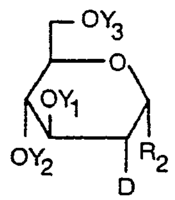

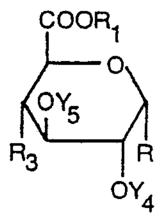 (VI)

Dans ces formules, $R_2$ et $R_3$ représentent deux groupements réactifs autorisant l'établissement, sélectivement, d'une liaison 1,4α, ces groupements étant choisis parmi ceux compatibles avec les autres groupements des monosaccharides;

— $Y_1$ à $Y_5$ représentent des groupements de bloquage permettant, de par leur nature, d'introduire successivement les groupements fonctionnels désirés sans que les groupements de bloquage restant ne soient affectés, et

— D représente un groupement azoté précurseur d'un groupement fonctionnel azoté —NHB tel que défini ci-dessus, de préference un groupe azide;

— R et $R_1$ présentent les significations données ci-dessus.

Selon une disposition de grand intérêt de l'invention, le groupement de bloquage $Y_3$, qui occupe une position destinée à être sulfatée, est constitué par un groupement permettant de réaliser sélectivement l'opération de sulfatation sans affecter les autres groupements. De manière préférée, $Y_3$ représente un groupe acétyle. Le choix d'un groupement de ce type, plus spécialement d'un groupement acétyle, sera également effectué, en cours de synthèse, pour d'autres positions destinées à être sulfatées, à savoir pour *B* dans —NHB.

Conformément à une autre disposition, les groupements de bloquage $Y_1$, $Y_2$, $Y_4$ et $Y_5$ qui seront éliminés en cours de synthèse pour libérer des groupes hydroxyle sont constitués par des groupes benzyle.

Dans un mode préféré de réalisation de l'invention, la réaction de condensation des monosaccharides V et VI est basée sur la réaction d'un halogénure avec une fonction alcool.

On met alors avantageusement en oeuvre un monosaccharide V dans lequel $R_2$ représente un halogénure, de préference un bromure, ou encore un chlorure, avec um monosaccharide VI dans lequel $R_3$ représente un groupe hydroxyle.

Cette réaction est avantageusement réalisée en milieu solvant, en particulier dans un solvant organique du type du dichlorométhane. On utilise avantageusement un catalyseur, tel que du triflate d'argent et également un accepteur de protones tel que de la sym-collidine.

On traite ensuite le disaccharide à liaison 1,4α obtenu, de structure:

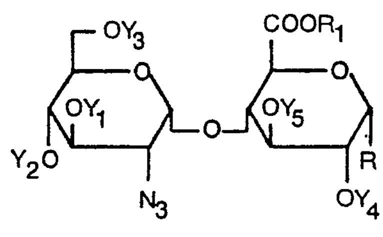 (VII)

de manière à introduire sélectivement et successivement les groupements fonctionnels désirés.

Afin d'introduire un groupe sulfate à la place de $Y_3$, on soumet tout d'abord le disaccharide VII à une réaction d'hydrolyse sous l'action d'une base forte telle que de la soude, puis à l'action d'un agent d'alcoylation afin de maintenir comme substituant A, un groupe $—COOR_1$.

Le disaccharide obtenu, qui comporte ainsi une fonction alcool primaire $—CH_2OH$ en position 6 du motif D-glucosamine, est soumis à l'action d'un agent de sulfatation dans des conditions permettant de remplacer le groupe $—CH_2OH$ en position 6 par un groupe sulfate sans affecter les autres groupes de bloquage de la molécule ainsi que le groupe azide. On utilise avantageusement, à cet effet, un complexe de

triméthylamine et de $SO_3$. L'introduction du cation $M_1$ désiré peut être notamment réalisée à l'aide d'une résine échangeuse d'ions comprenant ce cation, ou encore, après passage sous forme acide, par neutralisation avec la base du cation.

Au cours d'une étape suivante, on procède alors avantageusement à la libération des groupes —OH bloqués par $Y_1$, $Y_2$, $Y_4$ et $Y_5$ et à la transformation du groupe $—N_3$ en groupe $—NH_2$.

A cet effet, il est approprié de recourir à une réaction d'hydrogénation, avec de l'hydrogène en présence d'un catalyseur, en opérant dans des conditions compatibles avec le maintien du groupement sulfate en position 6 du motif D-glucosamine.

Selon une variante de réalisation de l'invention, on prépare un disaccharide comportant un motif N-acétyl D-glucosamine en traitant le disaccharide précédemment obtenu avec un agent d'acétylation, en particulier de l'anhydride acétique.

On opère avantageusement en milieu faiblement basique, à un pH de l'ordre de 8 dans des conditions n'affectant pas les autres substituants du disaccharide.

Selon une autre variante de réalisation de l'invention, on prépare un disaccharide comportant un motif N-sulfate D-glucosamine en traitant le disaccharide obtenu après l'étape d'hydrogénation avec un agent de sulfatation.

Il apparaît approprié d'effectuer cette réaction à pH basique entre 9 et 10 environ, avantageusement à l'aide d'un complexe de trimèthylamine et de $SO_3$. Pour obtenir ce groupe sulfate, sous forme de sel, on utilise avantageusement une résine échangeuse d'ions contenant le cation que l'on souhaite introduire, ou encore, après passage sous forme acide, on neutralise avec la base du cation.

Le traitement des produits N-acétylé ou N-sulfatés évoqués ci-dessus par un hydroxyde métallique permet d'obtenir, ci souhaité, les disaccharides correspondant comportant un groupe carboxylate en position 6 du motif glucuronique. On utilise avantageusement de la soude pour former du carboxylate de sodium.

Les disaccharides intermédiaires sont des produits nouveaux et en tant que tels entrent également dans le cadre de l'invention. Il en est de même du monosaccharide (2) dècrit dans les exemples.

D'autres caractéristiques et avantages de l'invention apparaîtront dans la description des exemples quid suivent et en ce se reportant aux figures dans lesquelles

— les figures 1 et 2 représentent l'ensemble du schéma réactionnel correspondant aux synthèses décrites dans les exemples, et

— les figures 3 et 4 les spectres RMN de disaccharides de l'invention.

Les abréviations utilisées dans les formules présentent les significations suivantes:

Ac représente un groupe acétyle,

Bn un groupe benzyle et

Me un groupe méthyle.

Les composés des figures 1 et 2 sont identifiés par des références numériques entre parenthèses, qui sont également utilisées dans les exemples pour les désigner.

Exemple 1

Préparation du (méthyl 2,3-di-O-benzyl-α-D-glucopyranoside) uronate de méthyle.

Dans 20 ml de chlorure de méthylène, on soumet à agitation pendant une nuit dans l'obscurité, un mélange de 0,75 g (2 mmole) de 2,3-di-O-benzyl-α-D-glucopyranoside de méthyle, 20 mg (0,08 mmole) de dimethyl-aminopyridine, 0,6 ml (4 mmole) de triéthylamine et 0,84 g (3 mmole) de chlorure de trityle.

Une chromatographie en couche mince (méthanol/chloroforme: 0,2/20, v/v) montre que la réaction est complète (le monosaccharide de départ est préparé selon la méthode de Freudenberg K. et Plankenhorn E, décrite dans Ber. Deut. Chem. Ges. *73* (1940) 621—631.

On ajoute successivement à ce mélange 0,6 ml (4 mmole) de triéthylamine, 0,35 ml (3 mmole) de chlorure de benzoyle, 50 mg(0,20 mmole) de diméthylaminopyridine et on prolonge l'agitation pendant deux jours.

Pour éliminer le groupement trityle, on ajoute au mélange une solution de 0,5M d'acide paratoluène sulfonique dans du méthanol (20 ml).

Au bout d'une heure, on dilue le mélange avec du chlorure de méthylène, puis on lave avec de l'eau jusqu'à neutralité. On purifie le produit par chromatographie sur colonne (40 g) en utilisant un mélange éther-hexane (3/1, v/v).

On obtient une mousse blanche pure (0,84 g; 87,7%) qu'on oxyde immédiatement selon la méthode de Kovac P., Alföldi J., Kosik M. décrite dans Chem. Zvesti *28* (1974) 820—832.

On dissout 480 mg (1 mmole) du produit dans 8 ml d'acétone et on ajoute à 0°C à la solution refroidie, sous agitation, une solution d'oxyde de chrome VI dans de l'acide sulfurique 3,5 M (1,15 ml de la solution de chrome contient 1,17 g dans 5 ml).

On laisse le mélange se réchauffer à température ambiante, puis au bout de deux heures on ajoute de la glace et de l'eau et on extrait le produit avec du chloroforme.

La phase chloroformique est lavée avec de l'eau et le solvant séché est évaporé.

On dissout la mousse obtenue dans du méthanol (10 mg/ml) et on ajoute de la soude (5 ml d'une solution 3M).

Après trois heures, on lave la phase aqueuse deux fois avec de l'éther puis on l'acidifie avec de l'acide

chlorydrique. On extrait alors le produit avec de l'éther. On lave la phase organique avec de l'eau, on la séche et on la concentre.

La solution ainsi obtenue est méthylée avec du diazométhane dans de l'éther et purifié sur une colonne de gel de silice (15 g) dans un mélange éther-hexane (2/1); v/v).

Rendement: 233 mg, 57,9%.

Le composé est cristallisé dans un mélange hexane-éther et présente les caractéristiques suivantes:

P.F.: 82°C;

$[\alpha]_D^{20}$: +17,5 (c = 1- chloroform);

Spectre $^1$H RMN ($Me_4$ Si; référence interne) δ 2,9 (d, 1H, O<u>H</u>) 3,35 (s, 3H, OC<u>H</u>$_3$) 3,69 (s, 3H, $COOCH_3$) 7,27 (12H, 2 Ph).

Analyse:

| | | |
|---|---|---|
| calculé pour $C_{22}H_{26}O_7$: | C, 65,65; | H, 6,51. |
| trouvé: | C, 65,53; | H, 6.29. |

Exemple 2

Préparation du disaccharide (3)

La synthèse de ce disaccharide (3) est réalisée à partir des monosaccharides (1) et (2) en opérant comme suit. On effectue la réaction à 0°C sous azote et à l'abri de la lumière.

A une solution du composé (2) (40 mg) dans le dichlorométhane sec (2 ml), on ajoute successivement du composé (1) (98 mg) de la sym-collidine (35 µl) et du triflate d'argent (56,5 mg). Après une heure trente, le mélange réactionnel est dilué par du dichlorométhane (50 ml). La solution est filtrée, lavée avec du bicarbonate de sodium saturé (deux fois 20 ml), puis avec de l'eau et enfin séchée.

Le sirop obtenu après concentration est chromatographié sur gel de silice (15 g) dans le système acétate d'éthyle/hexane (1,3, v/v).

On obtient ainsi le disaccharide (3) (60,2 mg; 74;).

Le spectre de RMN confirme la structure recherchée: signaux observés (par rapport à $Me_4Si$, standard interne), δ 1,95 (s, 3H, —OCOC<u>H</u>$_3$) 3,34 (s, 3H, OC<u>H</u>$_3$) 3,68 (s, 3H, CO—O—C<u>H</u>$_3$) 5,53 (d, 1H, 3,5 Hz, H'—1) 7,20—7,40 (m, 20 H, 4 Ph).

Exemple 3

Préparation du disaccharide (6)

Dans une première étape a), on effectue une saponification du groupement —OAC en position 6 du motif D-glucosamine, puis on procède dans une étape b) à la sulfatation du groupe —OH en position 6, et au cours d'une étape c) d'hydrogénation on élimine les groupements Bn de bloquage et on transforme simultanément le groupe —$N_3$ en groupe —$NH_2$.

*a — préparation du disaccharide (4) par saponification —*

On dissout le composé (3) dans du méthanol (10 ml), puis on ajoute de la soude 1 N (2 ml). Après trois heures, la solution est neutralisée par passage sur une résine Dowex 50 W × 4 $H^+$ (5 ml). Après concentration, le résidu est méthylé par du diazométhane, afin de réintroduire le groupe méthyle éliminé en même temps que l'acétate, on obtient, ainsi le composé (4).

*b — préparation du disaccharide (5) par sulfatation —*

Le composé (4) dissous dans du DMF anhydre (3 ml) On ajoute ensuite le complexe triméthylamine/$SO_3$ (20 mg) et on laisse à 65°C. Après une nuit, le mélange est évaporé à sec, repris par du chloroforme, dilué avec du méthanol, puis passé sur une colonne de résine DOWEX 50—$Na^+$. La phase organique est lavée à l'eau, séchée, puis le chloroforme est évaporé. On obtient le composé (5) (40 mg).

*c — préparation du disaccharide (6) par hydrogénation —*

Le composé (5) est dissous dans du méthanol (10 ml) et de l'eau (1 ml). On ajoute 40 mg de Pd/C à 5% et on soumet à l'action de l'hydrogène pendant 48 heures. Après filtration et évaporation, on obtient le composé (6) (29 mg).

Exemple 4

Préparation des disaccharides (7) et (8)

On prépare ces dérivés en soumettant le disaccharide (6) à une réaction d'acétylation du groupe —$NH_2$ du motif à structure glucosamine, suivie d'une saponification du groupe —COOMe du motif à structure acide uronique. Le composé (6) (14 mg) est dissous dans du méthanol (3 ml) Le pH est ajusté à 8 avec de la soude 1N. On ajoute alors de l'anhydride acétique (100 µl). Après 30 minutes, on évapore à sec. Le résidu est dissous dans de l'eau (1,5 ml) et de la soude 1 N est ajoutée (0,5 ml). Après une nuit à température ambiante, la solution est neutralisée à l'aide d'acide chlorhydrique. Le produit est ensuite dessalé par passage sur une colonne de Séphadex G—25 (1,8 × 20 cm). Les fractions contenant le produit sont rassemblées et chromatographiées sur une résine échangeuse d'anions (AG 1 × 2 200—400 mesh; lit de 1 ml). Les produits sont élués à l'aide d'un gradient de chlorure de sodium (0→3M). Après regroupement des fractions contenant le composé (8), celui-ci est dessalé par passage sur la colonne de Séphadex G—25 utilisée ci-dessus. m = 3,4 mg.

La structure du composé (8) est confirmée par dosage de l'acide uronique, de la glucosamine et des sulfates et par son spectre RMN (Voir figure 3) (Les significations des symboles utilisés sur cette figure sont les suivantes.

G désigne le signal du carbone anomère en 1 de l'acide uronique et Aa—1 celui de la N-acétyl glucosamine; Aa—6 celui du groupe O-sulfate en position 6 de la glucosamine sulfatée; A—a2 celui du carbone en position 2 du motif glucosamine; OMe, celui du groupe méthylglucoside et MeOH, celui du méthanol servant de standard interne). Son spectre U.V. présente un maximum d'absorption à 205 nm.

Exemple 5

Préparation des disaccharides (9) et (10)

Pour obtenir ces dérives, on soumet tout d'abord le disaccharide (6) à une réaction de sulfatation pour transformer le groupe $-NH_2$ en groupe $-NHSO_3Na$, puis à une réaction desaponification du groupe —COOMe comme indiqué ci-dessus.

Le produit (6) (14 mg) est dissous dans l'eau (5 ml).

Le pH est amené et maintenu à 9,5 à l'aide d'addition, contrôlée automatiquement, de soude 0,1N. On ajoute du complexe triméthylamine/$SO_3$ (20 mg). Après une nuit, on procède à une nouvelle addition de complexe (30 mg). Après 24 heures, on ajoute de la soude 1N (1 ml) et on laisse 1 heure à température ambiante. Après passage sur résine échangeuse de cations (Dowex 50 W $H^+$), puis neutralisation par la soude, le composé (10) est purifié comme décrit pour le composé (8) (dessalage, échange d'ions, dessalage). On obtient ainsi 2,9 mg du produit (10).

La structure du produit 10 est confirmée par dosage de l'acide uronique, de la glucosamine et des sulfates ainsi que par son spectre RMN (voir figure 4). Sur cette figure, G, OMe, MeOH présentent les significations utilisées pour la figure 3, As—1 représente le signal du carbone anomère en position 1 de la glucosamine N-sulfatée, As—6 celui du carbone en position 6 de la glucosamine sulfatée et As—2 celui du carbone anomère de la glucosamine N-sulfatée en position 2.

**Revendications**

1. Disaccharides 1,4α formés de motifs à structure D-glucosamine et acide glucuronique, caractérisés en ce qu'ils répondent à la formule (I):

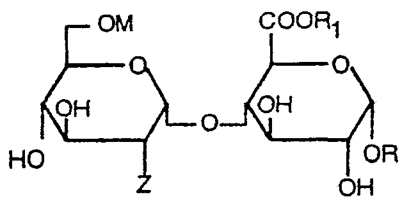

(I)

dans laquelle:

— *Z* représentent un groupe azide $N_3$ ou un groupe comportant une fonction amine, pouvant être obtenue selon les réactions classiques de chimie organique, à partir du groupe azide;

— *M* représente un atome d'hydrogène ou un groupe $-SO_3M_1$ dans lequel $M_1$ représente un cation organique ou minéral, et, dans ce dernier cas, en particulier un métal alcalin, ou représente un groupe acétyle;

— *R* représente un radical alcoyle de 1 à 4 atomes de carbone, en particulier, un radical méthyle, ou encore aryle, et

— $R_1$ représente un atome d'hydrogène, un radical alcoyle comportant de 1 à 4 atomes de carbone, en particulier, un radical méthyle, ou un métal, en particulier, un métal alcalin.

2. Disaccharides selon la revendication 1, caractérisées en ce que *Z* représente un groupe de structure —NHB dans laquelle *B* représente un atome d'hydrogène ou un groupe acétyle ou un groupe sulfate éventuellement sous forme de sel avec un cation organique ou minéral, et, dans ce dernier cas, en particulier un cation alcalin.

3. Disaccharides selon la revendication 1, caractérisées en ce qu'ils répondent à la formule II:

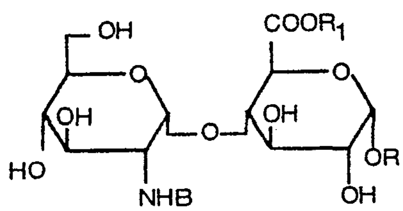

(II)

ou à la formule III:

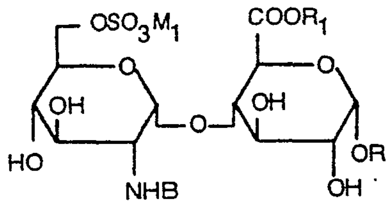

(III)

dans lesquelles, *B*, *R*, $R_1$ et $M_1$ présentent les significations données ci-dessus, $M_1$ représentant avantageusement un métal alcalin, en particulier, du sodium.

4. Disaccharides selon la revendication 3, caractérisé en ce que *B* représente un groupe acétyle.

5. Disaccharides selon la revendication 3, caractérisés en ce que *B* représente un groupe $—SO_3M_2$ dans lequel $M_2$, identique ou différent de $M_1$ défini ci-dessus, représente un métal alcalin, en particulier du sodium.

6. Disaccharides selon la revendication 4 ou 5, caractérisés en ce que *R* et $R_1$ sont identiques et représentent un groupe alcoyle, en particulier méthyle ou bien *R* et $R_1$ sont différents, $R_1$ représentant un métal alcalin, en particulier du sodium et *R* un radical alcoyle en particulier méthyle.

7. Disaccharide caractérisé en ce qu'il répond à la formule IV:

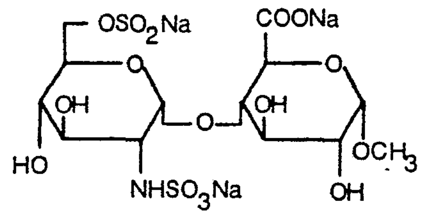

(IV)

ce disaccharide présentant une activité anti-Xa (Yin-Wessler) de 1000 à 2000 µ/g.

8. Médicaments, caractérise en ce qu'ils comprennent une quantité efficace d'au moins un disaccharide selon l'une quelconque des revendications 1 à 7.

9. Médicaments selon la revendication 8, caractérisés en ce qu'ils sont administrés par voie orale, rectale ou sous forme injectable.

10. Utilisation des disaccharides selon l'une des revendications 1 à 7 comme réactifs biologiques.

11. Procédé de préparation de disaccharides selon l'une quelconque des revendications 1 à 7, caractérisé en ce qu'

on met en oeuvre deux monosaccharides répondant respectivement aux formules V et VI:

(V)

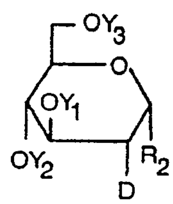

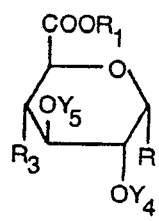

(VI)

dans lesquelles $R_2$ et $R_3$ représentent deux groupements réactifs autorisant l'établissement, sélectivement, d'une liaison 1,4α, ces groupements étant choisis parmi ceux compatibles avec les autres groupements des monosaccharides;

— $Y_1$ à $Y_5$ représentent des groupements de blocage permettant de par leur nature d'introduire successivement les groupements fonctionnels désirés sans que les groupements de blocage restant ne soient affectés, et

— D représente un groupement azoté précurseur d'un groupement fonctionnel azoté —NHB tel que défini ci-dessus, de préférence un groupe azide;

— R et $R_1$ présentent les significations données ci-dessus,

— on réalise une réaction de condensation entre ces deux monosaccharides afin d'établir la liaison 1,4α recherchée, en opérant dans des conditions compatibles avec les différents substituants des monosaccharides, ce qui conduit à un disaccharide de formule VII:

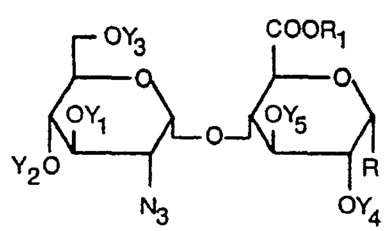

(VII)

— on procède par étapes successives à l'introduction des groupements fonctionnels désirés pour un disaccharide donné par mise en jeu de réaction spécifiques correspondantes,

— on libère les groupes hydroxyle des positions 2 et 3 du motif uronique et des positions 3 et 4 du motif glucosamine et on transforme le groupe —$N_3$ en groupe $NH_2$ en soumettant le disaccharide à une étape d'hydrogénation, et le cas échéant,

— on soumet le disaccharide ainsi obtenu à l'action d'un agent d'acétylation ou de sulfatation pour transformer le groupe —NH2 en groupe —N-acétyl ou —N-sulfate, ce dernier étant obtenu, le cas échéant sous forme de sel, et, si on le souhaite

— on soumet le disaccharide résultant à l'action d'un hydroxyde métallique pour former le carboxylate correspondant en position du motif uronique.

12. Procédé selon la revendication 11, caractérisé en ce que $Y_3$ est constitué par un groupement permettant d'introduire ultérieurement, dans cette position, un groupe sulfate et représente plus particulièrement un groupe acétyle.

13. Procédé selon l'une quelconque des revendications 11 ou 12, caractérisé en ce que les groupements de blocage $Y_1$, $Y_2$, $Y_4$ et $Y_5$ qui seront éliminés en cours de synthèse pour libérer des groupes hydroxyle, sont constitués par des groupes benzyle.

14. Procédé selon l'une quelconque des revendications 11 à 13, caractérisé en ce que $R_2$ représente un halogénure, de préférence un bromure, ou encore un chlorure, et $R_3$ un groupe alcool.

15. Procédé selon l'une quelconque des revendications 11 à 14, caractérisé en ce qu'on soumet tout d'abord le disaccharide VII à une réaction d'hydrolyse sous l'action d'une base forte telle que de la soude puis à l'action d'un agent d'alcoylation afin de maintenir comme substituant A, un groupe —$COOR_1$, qu'on soumet, le cas échéant, le disaccharide obtenu, comportant une fonction alcool primaire —$CH_2OH$ en position 6 du motif D-glucosamine, à l'action d'un agent de sulfatation dans des conditions permettant de remplacer le groupe —$CH_2OH$ en position 6 par un groupe sulfate sans affecter les autres groupes de bloquage de la molécule ainsi que le groupe azide, un complexe de triméthylamine et de $SO_3$ étant avantageusement utilisé pour introduire un groupe —$SO_3Na$, qu'on effectue ensuite, le cas échéant, une réaction d'hydrogénation pour libérer les groupes —OH bloqués par $Y_1$, $Y_2$, $Y_4$ et $Y_5$ et transformer le groupe —$N_3$ en groupe —$NP_2$ et que (si on le désire), pour préparer un disaccharide comportant un motif N-acétyl D-glucosamine on traite le disaccharide précédemment obtenu avec un agent d'acétylation, en particulier de l'anhydride acétique, et pour préparer un disaccharide comportant un motif N-sulfate D-glucosamine, on traite le disaccharide obtenu après l'étape d'hydrogénation avec un agent de sulfatation, ces réactions étant suivies, si souhaité, d'un traitement avec un hydroxyde métallique afin d'obtenir un disaccharide comportant un groupe carboxylate en position 6 du motif glucuronique, avantageusement avec de la soude pour former un groupe —COONa.

16. Produits intermédiaires dans le procédé selon la revendication 11, caractérisé en ce qu'il s'agit des disaccharides suivants:

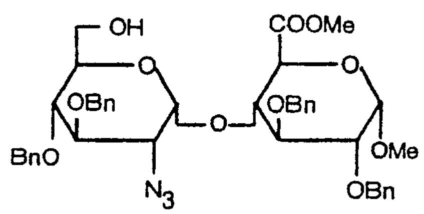

(4)

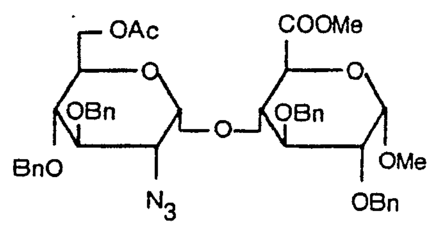

(5)

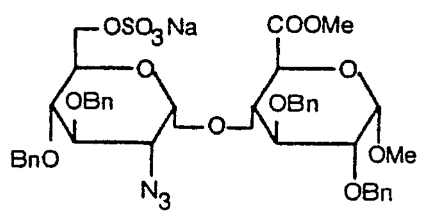

(6)

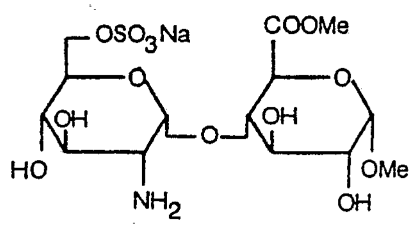

(7)

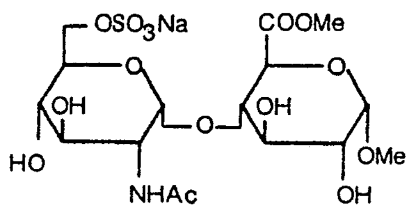

(8)

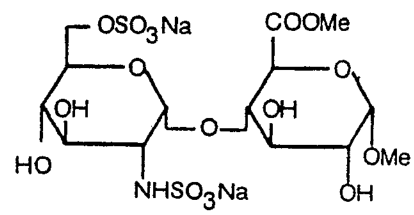

(9)

**Patentansprüche**

1. 1,4-α-Disaccharide aus Einheiten mit D Glucosamin- und Glucuronsäurestruktur, dadurch gekennzeichnet, daß sie der Formel (I):

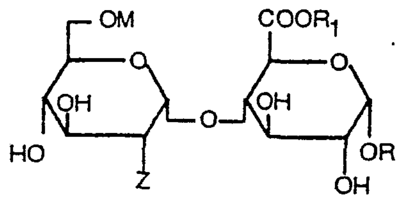

(I)

entsprechen, worin:

— Z eine Azidgruppe $N_3$ oder eine eine Aminfunktion aufweisende Gruppe darstellt, die aus der Azidgruppe nach den klassischen Reaktionen der organischen Chemie erhalten werden kann;

— M ein Wasserstoffatom oder eine Gruppe —$SO_3M_1$ darstellt, worin $M_1$ ein organisches oder mineralisches Kation und im letztgenannten Fall insbesondere ein Alkylimetall bedeutet, oder für eine Acetylgruppe steht;

— R einen Alkylrest mit 1 bis 4 Kohlenstoffatomen, insbesondere einen Methylrest, oder auch Aryl bedeutet, und

— $R_1$ ein Wasserstoffatom, einen Alkylrest mit 1 bis 4 Kohlenstoffatomen, insbesondere einen Methylrest, oder ein Metall, insbesondere ein Alkalimetall bedeutet.

2. Disaccharide nach Anspruch 1, dadurch gekennzeichnet, daß Z eine Gruppe mit der Struktur —NHB bedeutet, worin B ein Wasserstoffatom oder eine Acetylgruppe oder eine Sulfatgruppe, gegebenenfalls in Form eines Salzes mit einem organischen oder mineralischen Kation und im letztgenannten Fall insbesondere mit einem Alkalikation, darstellt.

3. Disaccharide nach Anspruch 1, dadurch gekennzeichnet, daß sie der Formel II:

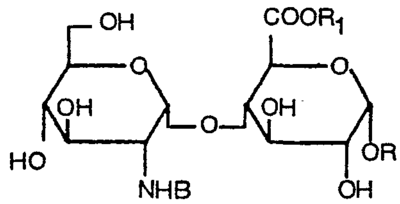

(II)

oder der Formel III:

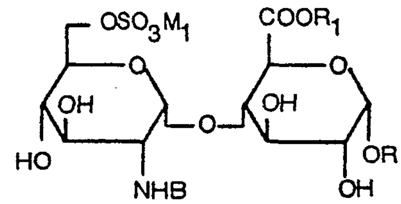

(III)

entsprechen, worin B, R, $R_1$ und $M_1$ die vorstehend angegebenen Bedeutungen aufweisen, wobei $M_1$ vorteilhaft ein Alkalimetall, insbesondere Natrium bedeutet.

4. Disaccharide nach Anspruch 3, dadurch gekennzeichnet, daß B eine Acetylgruppe darstellt.

5. Disaccharide nach Anspruch 3, dadurch gekennzeichnet, daß B eine Gruppe —$SO_3M_2$ darstellt, worin $M_2$, das mit dem oben definierten $M_1$ gleich oder davon verschieden sein kann, ein Alkalimetall, insbesondere Natrium bedeutet.

6. Disaccharide nach Anspruch 4 oder 5, dadurch gekennzeichnet, daß R und $R_1$ gleich sind und eine Alkylgruppe, insbesondere Methyl, bedeuten oder daß R und $R_1$ verschieden sind, wobei $R_1$ ein Alkalimetall, insbesondere Natrium bedeutet und R einen Alkylrest, insbesondere Methyl darstellt.

7. Disaccharid, dadurch gekennzeichnet, daß es der Formel IV:

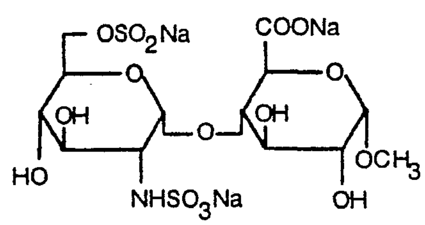

(IV)

entspricht, welches Disaccharid eine Anti-XA (Yin-Wessler)-Aktivität von 1000 bis 2000 Einheiten/Gramm aufweist.

8. Arzneimittel, dadurch gekennzeichnet, daß sie eine wirksame Menge wenigstens eines Disaccharids nach einem der Ansprüche 1 bis 7 enthalten.

9. Arzneimittel nach Anspruch 8, dadurch gekennzeichnet, daß sie auf oralem Wege, rektalem Wege oder in injizierbarer Form verabreicht werden.

10. Verwendung der Disaccharide nach einem der Ansprüche 1 bis 7 als biologische Reagenzien.

11. Verfahren zur Herstellung der Disaccharide nach einem der Ansprüche 1 bis 7, dadurch gekennzeichnet, daß man zwei Monosaccharide einsetzt, die den Formeln V bzw. VI:

(V) 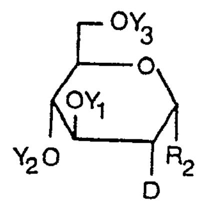

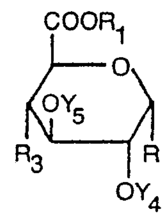 (VI)

entsprechen, worin $R_2$ und $R_3$ zwei reaktionsfähige Gruppen darstellen, welche die selektive Ausbildung einer 1,4-α-Bindung gestatten, wobei diese Gruppen unter jenen ausgewählt sind, die mit den anderen Gruppen der Monosaccharide verträglich sind;

— $Y_1$ bis $Y_5$ Schutzgruppen darstellen, die zufolge ihrer Natur die aufeinanderfolgende Einführung der gewüschten funktionellen Gruppen ohne Beeinträchtigung der verbleibenden Schutzgruppen gestatten, und

— D eine stickstoffhaltige Vorläufergruppe für eine stickstoffhaltige funktionelle Gruppe —NHB, wie vorstehend definiert, bedeutet, vorzugsweise eine Azidgruppe;

— R und $R_1$ die vorstehend angegebenen Bedeutungen aufweisen,

— daß man zwischen diesen beiden Monosacchariden eine Kondensationsreaktion ausführt, um die angestrebte 1,4-α-Bindung auszubilden, wobei man unter Bedingungen arbeitet, die mit den unterschiedlichen substituenten der Monosaccharide verträglich sind, was zu einem Disaccharid der Formel VII:

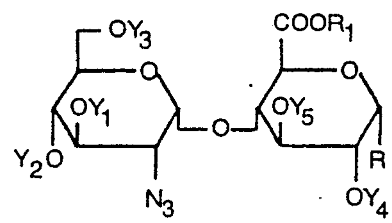 (VII)

führt,

— daß man in aufeinanderfolgenden Stufen die für ein bestimmtes Disaccharid gewünschten funktionellen Gruppen einführt, indem entsprechende spezifische Reaktionen ausgeführt werden,

— daß man die Hydroxylgruppen der 2- und 3-Stellungen der Uroneinheit und der 3- und 4-Stellungen der Glucosamineinheit freisetzt und die —$N_3$-Gruppe in eine $NH_2$-Gruppe überführt, indem man das Disaccharid einer Hydrierungsstufe unterwirft, und daß man in gegebenen Falle

— das solcherart erhaltene Disaccharid der Einwirking eines Acetylierungs- oder Sulfatierungsmittels unterwirft, um die —$NH_2$-Gruppe in eine —N-Acetylgruppe oder eine —N-Sulfatgruppe überzuführen, wobei die letztgenannte gegebenenfalls in Form eines Salzes erhalten wird, und daß man gewünschtenfalls

— das gebildete Disaccharid der Einwirkung eines Metallhydroxids unterwirft, um an der entsprechenden Stellung der Uroneinheit das Carboxylat auszubilden.

12. Verfahren nach Anspruch 11, dadurch gekennzeichnet, daß $Y_3$ aus einer Gruppe besteht, die an dieser Stellung die nachfolgende Einführung einer Sulfatgruppe gestattet und insbesondere eine Acetylgruppe bedeutet.

13. Verfahren nach Anspruch 11 oder 12, dadurch gekennzeichnet, daß die Schutzgruppen $Y_1$, $Y_2$, $Y_4$ und $Y_5$ die im Laufe der Synthese zur Freisetzung der Hydroxylgruppen beseitigt werden, aus Benzylgruppen bestehen.

14. Verfahren nach einem der Ansprüche 11 bis 13, dadurch gekennzeichnet, daß $R_2$ einen Halogenrest, vorzugsweise einen Bromrest oder auch einen Chlorrest, und $R_3$ eine Alkoholgruppe bedeuten.

15. Verfahren nach einem der Ansprüche 11 bis 14, dadurch gekennzeichnet, daß man zunächst das Disaccharid VII einer Hydrolysereacktion unter der Einwirkung einer starken Base wie Natronlauge und anschließend der Einwirkung eines Alkylierungsmittels unterwirft, um als Substituent A eine Gruppe —$COOR_1$ aufrecht zu erhalten, daß man im gegebenen Falle das erhaltene Disaccharid, das in der 6-Stellung der D-Glucosamineinheit eine primäre Alkoholfunktion aufweist, der Einwirkung eines Sulfatierungsmittels unter Bedingungen unterwirft, die einen Ersatz der Gruppe —$CH_2OH$ in der 6-Stellung durch eine Sulfatgruppe ohne Beeinträchtigung der anderen Schutzgruppen des Moleküls sowie der

Azidgruppe gestatten, wobei vorteilhaft ein Komplex aus Trimethylamin und $SO_3$ zum Einführen einer $-SO_3Na$-Gruppe verwendet wird, daß man anschließend gegebenenfalls eine Hydrierungsreaktion vornimmt, um die durch $Y_1$, $Y_2$, $Y_4$ und $Y_5$ blockierten —OH-Gruppen freizusetzen und die $-N_3$-Gruppe in die $-NH_2$-Gruppe überzuführen und daß man, falls dies gewünscht ist, zur Herstellung eines eine N-Acetyl-D-glucosamineinheit aufweisenden Disaccharids das zuvor erhaltene Disaccharid mit einem Acetylierungsmittel behandelt, insbesondere mit Essigsäureanhydrid, und zur Herstellung eines eine N-Sulfat-D-glucosamineinheit aufweisenden Disaccharids das nach der Hydrierungsstufe erhaltene Disaccharid mit einem Sulfatierungsmittel behandelt, an welche Reaktionen sich gewünschtenfalls eine Behandlung mit einem Metallhydroxid anschließt, um ein Disaccharid zu erhalten, das in der 6-Stellung der Glucuroneinheit eine Carboxylatgruppe aufweist, vorzugsweise eine Behandlung mit Natriumhydroxid zur Ausbildung einer Gruppe —COONa.

16. Zwischenprodukte im Verfahren nach Anspruch 11, dadurch gekennzeichnet, daß es sich um die folgenden Disaccharide handelt:

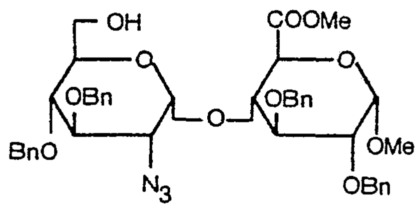

(4)

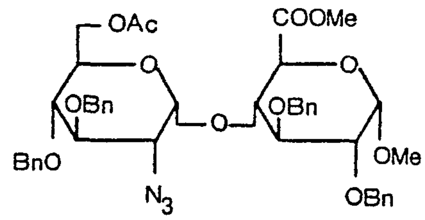

(5)

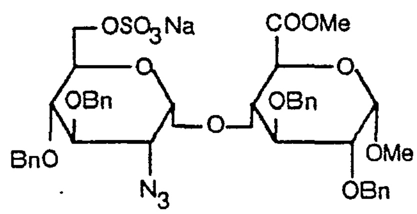

(6)

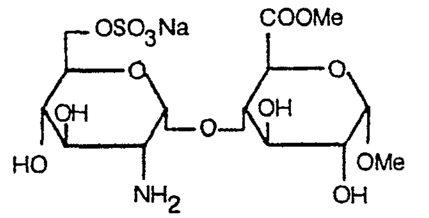

(7)

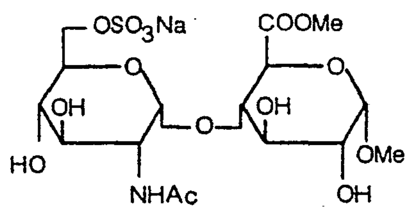

(8)

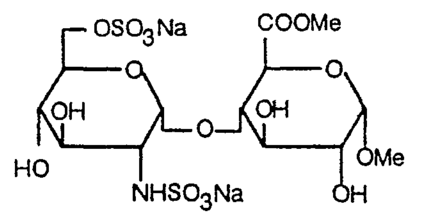

(9)

**Claims**

1. 1,4 α disaccharides formed from units with a D-glucosamine and glucuronic acid structure, characterised in that they correspond to the formula (I):

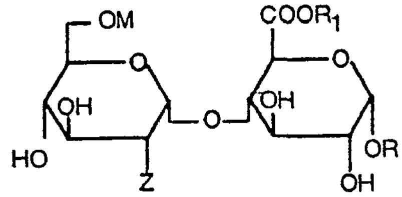

(I)

in which:

—*Z* represents an azide group or a group comprising an amino function, obtainable according to the usual reactions of organic chemistry, from the azido group,

—*M* represents a hydrogen atom or a $-SO_3M_1$ group in which $M_1$ represents an organic or inorganic cation, and, in the latter case, in particular an alkali metal, or represents an acetyl group;

— *R* represents an alkyl radical of 1 to 4 carbon atoms, in particular, a methyl radical or again aryl, and
— $R_1$ represents a hydrogen atom, an alkyl radical comprising from 1 to 4 carbon atoms, in particular, a methyl radical, or a metal, in particular, an alkali metal.

2. Disaccharides according to claim 1 wherein Z represents a group of the structure —NHB in which *B* represents a hydrogen atom or an acetyl group or a sulphate group possibly in the form of a salt with an organic or inorganic cation, and in the latter case, in particular an alkali cation.

3. Disaccharides according to claim 1, characterised in that they correspond to the formula II:

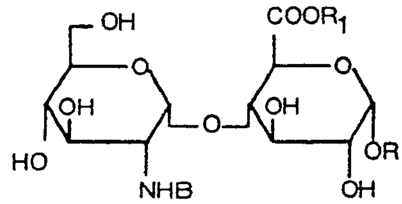

(II)

or to the formula III:

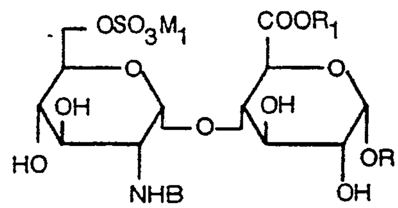

(III)

in which, *B, R, $R_1$* have the above-indicated meanings,
$M_1$ representing advantageously an alkali metal, in particular, sodium.

4. Disaccharides according to claim 3, wherein B represents an acetyl group.

5. Disaccharides according to claim 3, wherein *B* represents an —$SO_3M_2$ group in which $M_2$, identical or different from $M_1$ defined above, represents an alkali metal, in particular sodium.

6. Disaccharides according to claim 4 or 5, wherein *R* and $R_1$ are identical and represent an alkyl group, in particular methyl or indeed *R* and $R_1$ are different, $R_1$ representing an alkali metal, in particular sodium and *R* an alkyl radical in particular methyl.

7. Disaccharide characterised in that it corresponds to the formula IV:

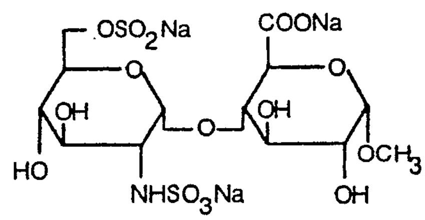

(IV)

this disaccharide having an anti-Xa activity (Yin-Wessler) of 1,000 to 2,000 μ/g.

8. Medicaments, characterised in that they comprise an effective amount of at least one disaccharide according to any one of claims 1 to 7.

9. Medicaments according to claim 8, characterised in that they are administered by oral or rectal route or under an injectable form.

10. Use of the disaccharides according to anyone of claims 1 to 7 as biological reagents.

11. Method of preparing disaccharides according to any one of claims 1 to 7, characterised by:
— using two monosaccharides having respectively formulae V and VI:

(V)

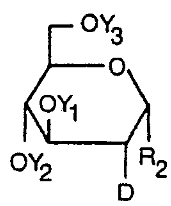

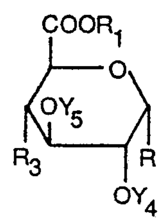

(VI)

in which $R_2$ and $R_3$ represent two reactive groups permitting the establishment, selectively, of a 1,4 α bond, these groups being selected from among those compatible with the other groups of the monosaccharides;
— $Y_1$ to $Y_5$ represent blocking groups enabling through their nature the introduction successively of the desired functional groups without the remaining blocking groups being affected, and

— D represents a nitrogenous group precursor of a nitrogenous functional group —NHB as defined above, preferably an azide group;

— R and $R_1$ have the above-indicated meanings,

— performing a condensation reaction between said two monosaccharides to establish the desired 1,4 α bond, under conditions compatible with the various substituants of the monosaccharides, which results in a disaccharide of formula VII:

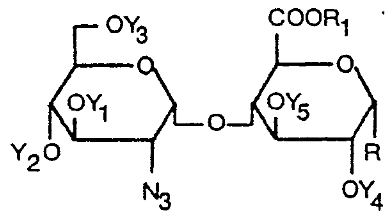

(VII)

— proceeding by successive steps with the introduction of the desired functional groups for a given disaccharide by bringing into play corresponding specific reactions,

— liberating the hydroxyl groups at positions 2 and 3 of the uronic unit and positions 3 and 4 of the glucosamine unit and converting the $—N_3$ group into a $—NH_2$ group by subjecting the disaccharide to an hydrogenation step and, optionally

— subjecting the resulting disaccharide to the action of an acetylation or a sulfation agent to convert the $—NH_2$ group into —N-acetyl or —N-sulfate group, the latter being optionally obtained under the salt form and, if desired,

— subjecting the resulting disaccharide to the action of a metallic hydroxide to form the corresponding carboxylate on the uronic unit.

12. Method according to claim 11, wherein $Y_3$ is constituted by a group enabling to further introduce at this position a sulfate group and more particularly represents an acetyl group.

13. Method according to any one of claims 11 or 12 wherein the blocking groups $Y_1$, $Y_2$, $Y_4$ and $Y_5$ which will be eliminated during the synthesis to liberate hydroxyl groups, are constituted by benzyl groups.

14. Method according to any one of claims 11 to 13 wherein $R_2$ represents a halide, preferably a bromide, or again a chloride, and $R_3$ an alcohol group.

15. Method according to any one of claims 11 to 14, wherein first the disaccharide VII is subjected to a hydrolysis reaction under the action of a strong base such as soda then to the action of an alkylating agent in order to maintain as substituent A, a $—COOR_1$ group, optionally subjecting the disaccharide obtained, comprising a primary alcohol function $—CH_2OH$ at the 6 position of the D-glucosamine unit, to the action of a sulphating agent under conditions enabling the replacement of the $—CH_2OH$ group at the 6 position by a sulphate group without affecting the other blocking groups of the molecule as well as the azide group, a complex of trimethylamine and $SO_3$ being advantageously used to introduce a $—SO_3Na$ group, then carrying out, as necessary, a hydrogenation reaction to liberate the —OH groups blocked by $Y_1$, $Y_2$, $Y_4$ and $Y_5$ and converting the $N_3$ group into a $—NH_2$ group and (if desired), to prepare a disaccharide comprising a D-glucosamine N-acetyl unit, treating the previously obtained disaccharide with an acetylating agent, in particular acetic anhydride, and to prepare a disaccharide comprising a D-glucosamine N-sulphate, the disaccharide obtained after the hydrogenation step is treated with a sulphating agent, these reactions being followed, is desired, by a treatment with a metal hydroxide in order to obtain a disaccharide comprising a carboxylate group at the 6 position of the glucuronic unit, advantageously with soda to form a —COONa group.

16. Intermediate compounds in the process according to claim 11 characterised in that they consist to the following disaccharides.

(4)

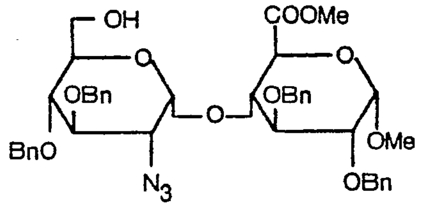

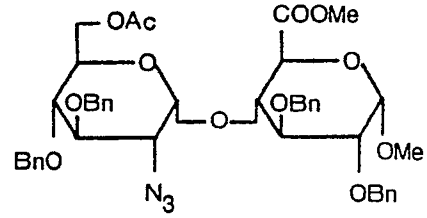

(5)

(6)

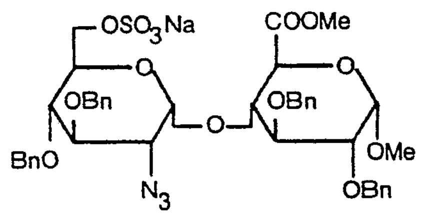

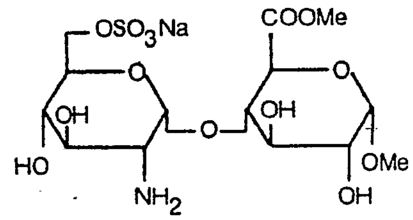

(7)

(8) 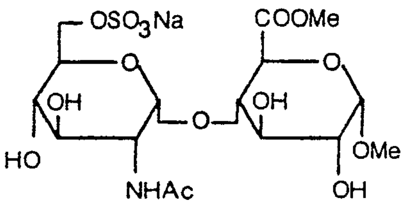

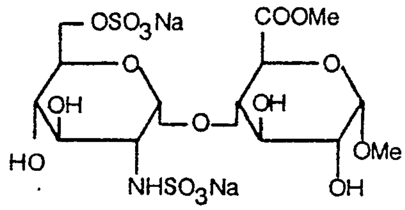 (9)

Fig.1.

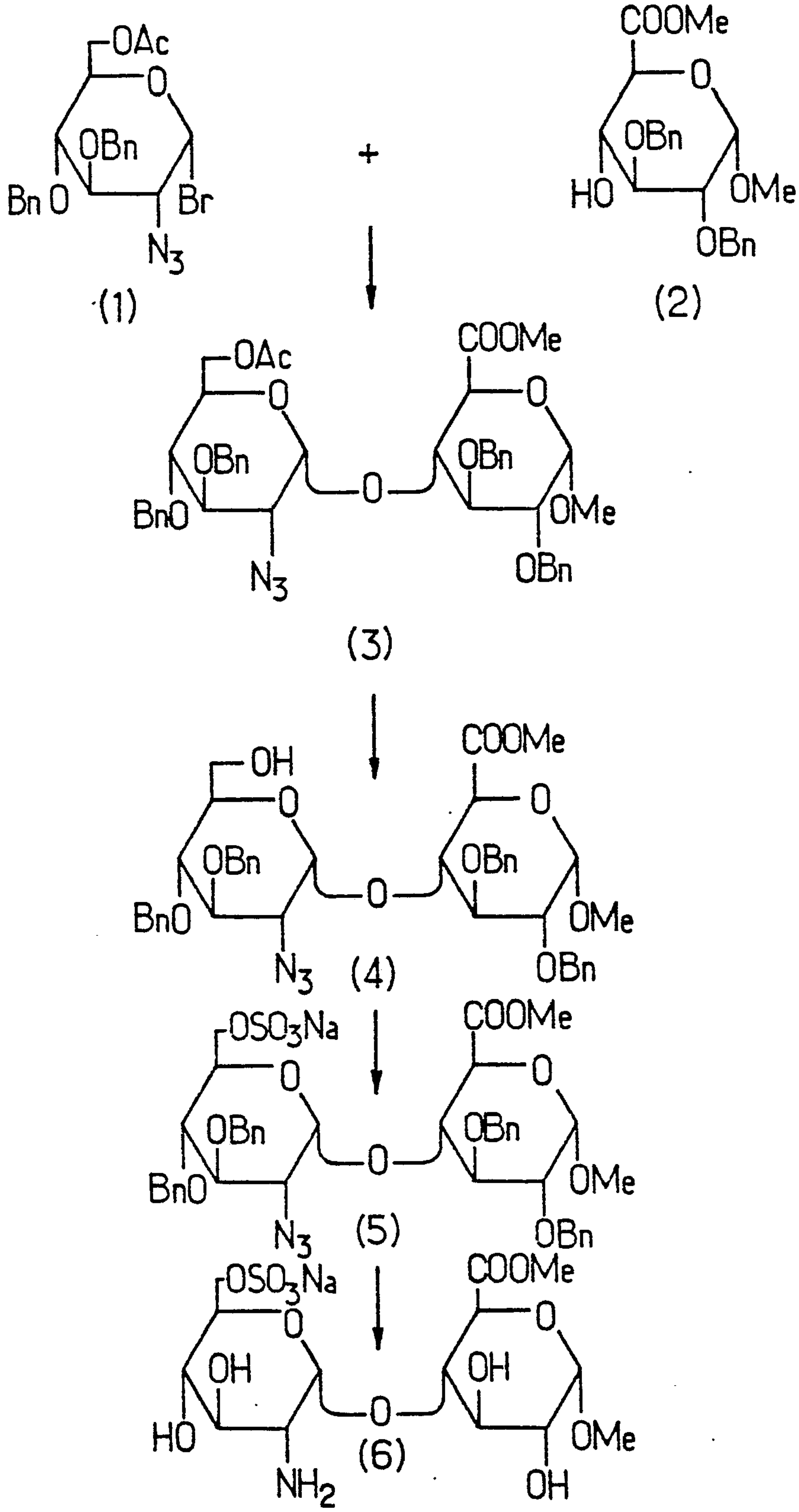

(1) (2) (3) (4) (5) (6)

Fig. 2.

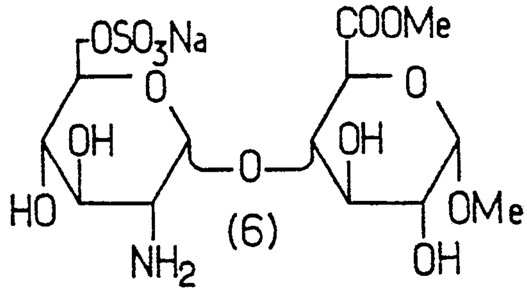

(6)

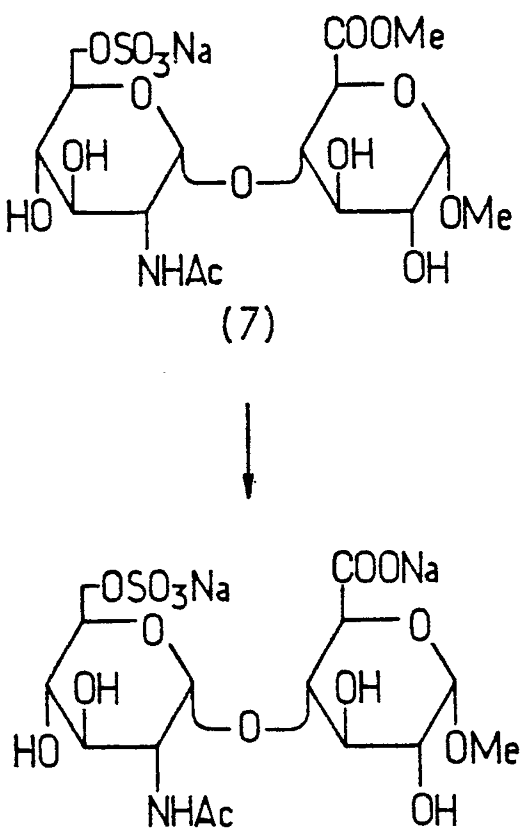

(7)

(8)

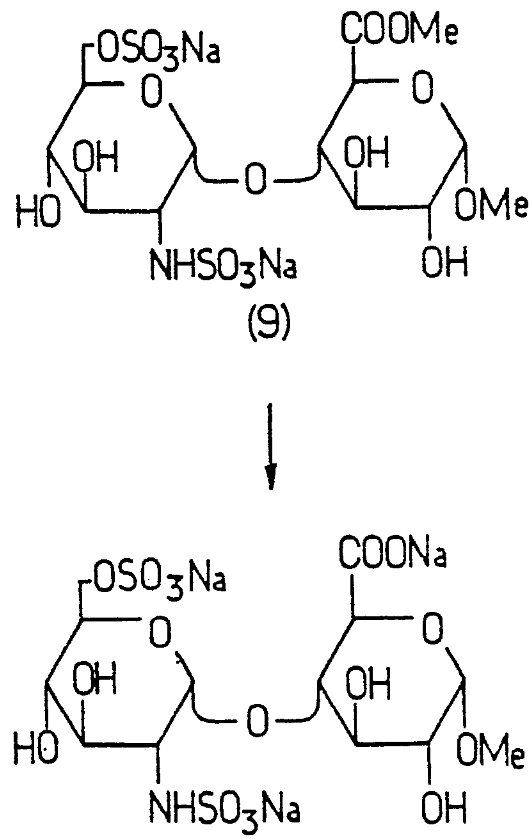

(9)

(10)

Fig.3.

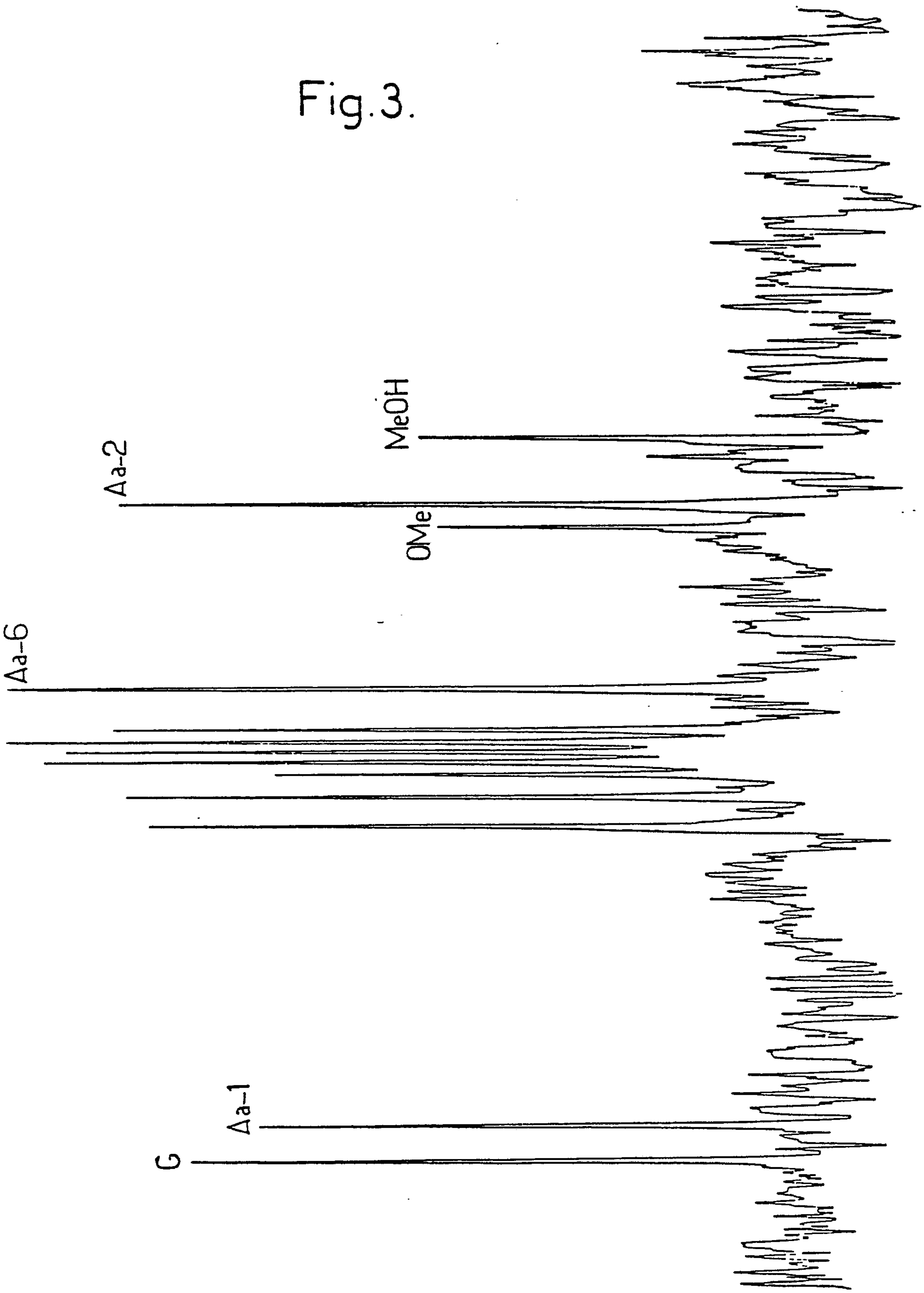

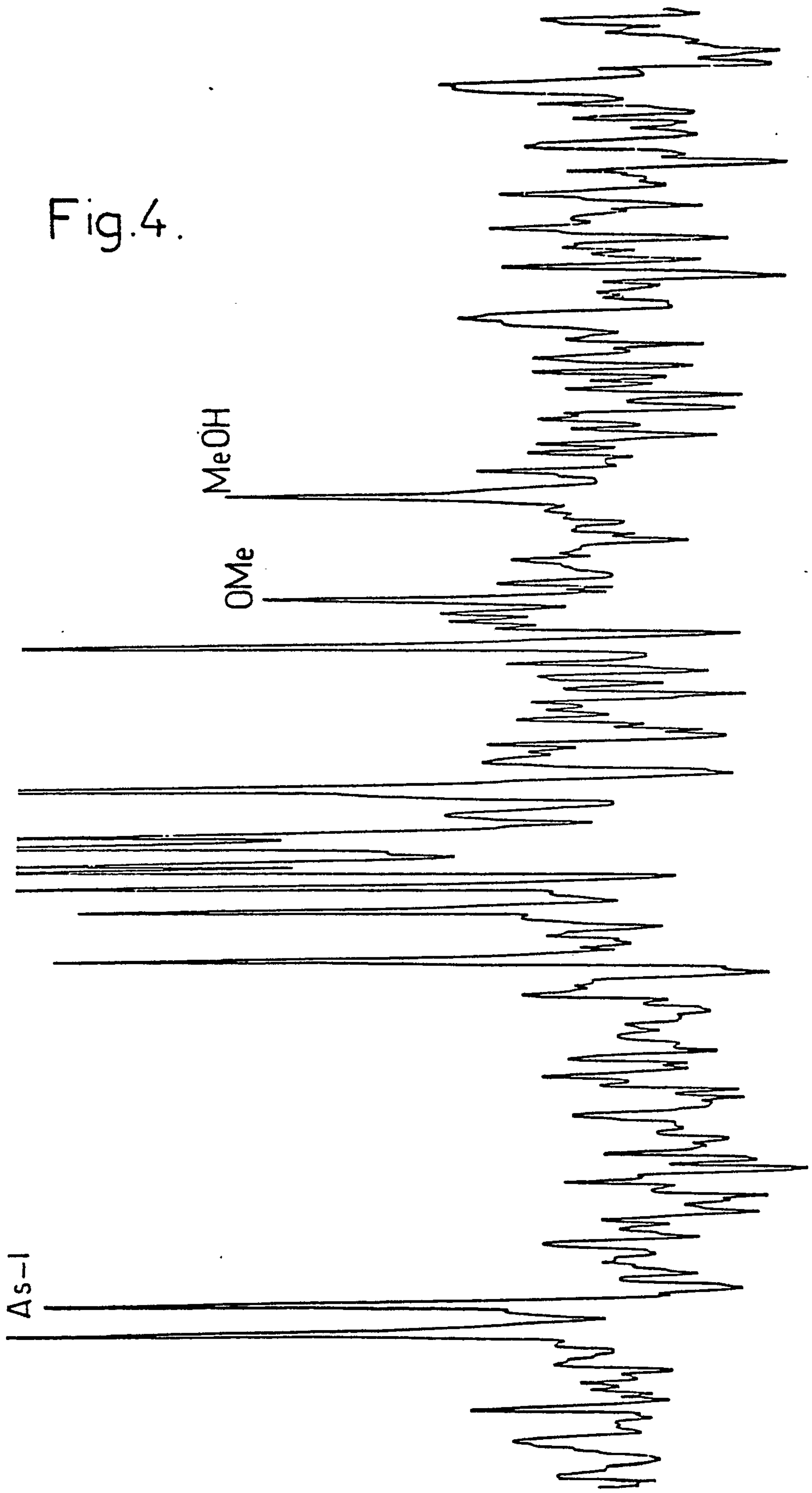


Fig.4.